# EUROPEAN PATENT SPECIFICATION

(11) **EP 3 740 267 B1**
(45) Date of publication and mention of the grant of the patent: **28.02.2024**
(21) Application number: 19700537.4
(22) Date of filing: 17.01.2019
(51) Int. Cl.: A61M 5/24, A61M 5/315, A61M 5/31

(54) **PACKAGING CONTAINER**
VERPACKUNGSBEHÄLTER
CONTENEUR D'EMBALLAGE

(30) Priority: 19.01.2018 EP 18305039
(43) Date of publication of application: 25.11.2020
(73) Proprietor: Sanofi, 75017 Paris (FR)
(72) Inventor: WEBER, Carsten, 65926 Frankfurt am Main (DE); WAGNER, Daniel, 65926 Frankfurt am Main (DE); FRICK, Désirée, 65926 Frankfurt am Main (DE); THIEL, Sebastian, 65926 Frankfurt am Main (DE); GACA, Sebastian, 65926 Frankfurt am Main (DE)
(74) Representative: Keil & Schaafhausen Patentanwälte PartGmbB
(86) International application number: PCT/EP2019/051177
(87) International publication number: WO 2019/141786

(56) References cited:
- WO-A1-2014/026721
- WO-A2-03/074103
- US-A1- 2017 232 196

## Description

The present invention is generally directed to a packaging container such as a cartridge or a pre-filled syringe and a drug delivery device comprising such packaging container.

The invention is directed to drug delivery devices in general including pen type drug delivery devices, autoinjectors and drug delivery devices comprising a pump drive, wherein the drug delivery device has a packaging container. The packaging container contains a usually liquid medicament formulation containing one or more pharmaceutically active compound and/or carrier forming the medicament for clinical and home treatment using such drug delivery devices.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. There are also so called fixed dose devices which only allow dispensing of a predefined dose without the possibility to increase or decrease the set dose.

There are different types of drug delivery devices delivering user variable doses: resettable devices (i.e., reusable) and non-resettable (i.e., disposable). For example, disposable pen delivery devices are supplied as self-contained devices comprising a primary packaging container. Such self-contained devices do not have removable pre-filled packaging containers. Rather, the pre-filled packaging containers may not be removed and replaced from these devices without destroying the device itself. Consequently, such disposable devices need not have a resettable dose setting mechanism. Additional application variants of devices are single dose and multi dose drug delivery devices. Emptying of drug delivery devices comprising a pump drive such as a durable pump or a patch pump may be realized by suction or pressure.

These types of delivery devices are generally comprised of three primary elements: a packaging container section that includes a packaging container, wherein the packaging container is usually a cartridge, often contained within a housing or holder; a needle assembly connected to one end of the packaging container section; and a dosing and/or actuating section connected to the other end of the packaging container section. A packaging container, e.g. a cartridge or ampoule, is a container which typically contains/includes a reservoir that is filled with a medicament formulation (e.g., insulin) with a movable rubber type plunger stopper (plug or bung) located at one end of the reservoir, and a top having a pierceable rubber seal located at the other, often necked-down, end. A crimped annular metal band is typically used to hold the rubber seal in place. The packaging container housing may be typically made of plastic material or glass. The packaging container is often referred to as primary packaging or primary container.

The needle assembly is typically a replaceable double-ended needle assembly. Before an injection, a replaceable double-ended needle assembly is attached to one end of the packaging container section, a dose is set, and then the set dose is administered. Such removable needle assemblies may be threaded onto, or pushed (i.e., snapped) onto the pierceable seal end of the packaging container.

The dosing and/or actuating section or dose setting mechanism or actuating mechanism is typically the portion of the pen device that is used to set a dose and/or to initiate and drive dose dispense. During an injection, a driving element such as a spindle, piston or piston rod of the dose setting mechanism presses against the plunger stopper (bung) of the packaging container and drives the plunger stopper into the direction of an attached needle assembly. This force causes the medication contained within the packaging container to be injected through the attached needle assembly. After an injection, as generally recommended by most drug delivery device and/or needle assembly manufacturers and suppliers, the needle assembly is removed and discarded.

For reusable drug delivery devices it is necessary to allow the piston rod or lead screw to be reset, i.e. pushed and/or wound back into the device, during the step of replacing an empty packaging container by a new (full) packaging container. In addition, many drug delivery devices comprise a dose limiter for preventing the setting of a dose, which exceeds the amount of liquid left in a packaging container of the drug delivery device. If such a dose limiter is provided, this dose limiter mechanism has to be reset, too.

In the following resetting of the device is to be understood the act of replacing or exchanging a packaging container involves a retraction of the piston rod or lead screw and, if present, bringing the dose limiter (last dose protection mechanism) back into an initial configuration allowing dose setting.

Interactions between a formulation and its packaging container can have a significant impact on the purity of the drug and the safety of the patients. With drug impurities and degradation among the primary causes of formulation recalls, management and control of associated risks is a key factor during formulation development and manufacturing processes.

During development of a new formulation for home treatment which is provided to the patient using a packaging container such as a cartridge a negative effect for the drug product formulation such as aggregation and formation of particles was observed.

Additionally, the break loose and gliding force properties of a packaging container are crucial for the performance of the system comprising the packaging container and the medicament formulation within the container during use.

To impact the frictional forces between a gasket and a cylinder of a dual chamber prefilled syringe, WO 03/074103 A2 therefore proposes to apply a coating to the gasket. Further, US 2017232196 A1 discloses a coating of a ferrule.

However, official authorities provide limits for break loose and gliding forces after manufacturing as well as after a pre-defined time the system is in storage as a quality and/or safety criteria. Further, the break loose and gliding force properties need to be enhanced due to new drive systems comprising a spring. Additionally, high speed filling of medicament formulation into a packaging container need to be provided as well.

Accordingly, it is an object of the present invention to provide a packaging container and a drug delivery device with above mentioned enhanced properties.

According to a first embodiment this object is solved by a packaging container, for example a cartridge or a primary container of or for a syringe or a pump device (including drug delivery devices comprising a pump drive), comprising a tubular housing and a plunger stopper (plug, bung) moveable within the housing. At least a section of an outer surface of the plunger stopper is in contact with an inner surface of the housing (e.g. a lateral surface). At least the section comprises a silicone-free coating which reduces break loose and/or gliding forces. This means that the silicone-free coating is provided at the outer surface of the plunger stopper which is in contact with an inner surface the housing and covers this surface at least partially.

The plunger stopper may have one circumferential section which is in contact with the inner surface of the housing or more than one such section at its lateral surface (lateral contact section) running circumferentially, for example two lateral contact sections or three lateral contact sections. Each such lateral contact section may be referred to as lamella, rib or sealing ring. Between at least two adjacent lateral contact sections the plunger stopper has a smaller diameter than in the lateral contact section and may there not be in direct contact with the inner surface of the housing. Each lateral contact section may be covered at least partially with the silicone-free coating. The length of each lateral contact section in longitudinal direction of the plunger stopper may be less than the full length of the plunger stopper in longitudinal direction, for example equal to or less than 50% of the full length of the plunger stopper, in another example equal to or less than 30% of the full length of the plunger stopper. The length of two lamellae of the same plunger stopper may be different.

The three-dimensional inner volume formed by the tubular housing and the plunger stopper contains the medicament formulation. The volume or inner space formed by the packaging container is closed on one end by the plunger stopper and may be closed on the other end by a seal, which is pierceable, for example, by a needle for discharging the medicament formulation. The seal is located at the end of the tubular housing which is opposite to the plunger stopper. The packaging container is used to encase a medicament formulation between the plunger stopper and the seal within the inner volume of the housing. The volume may be fully or partially filled by the medicament formulation. The silicone-free coating may be additionally provided at at least a second section of an outer surface of the plunger stopper, wherein the second section faces the inner volume of the packaging container. This surface forms an end surface of this volume and is therefore in contact with the medicament formulation encased within the packaging container. In one embodiment the whole area of the surface of the plunger stopper facing the inner volume is covered by the silicone-free coating. The silicone free coating at this surface of the plunger stopper functions as a barrier or protection layer for the material of the plunger stopper with respect to the medicament formulation contained within the volume.

In one embodiment the plunger stopper comprises a recess (bore) within its surface opposite the inner volume of the packaging container. The surface faces the driving element (e.g. piston, piston rod, spindle) of the dosing and/or actuating section. The recess may be formed conically and/or cylindrically and/or cuboid and may comprise a thread at its inner surface. The thread may serve for attachment of a piston rod or spindle to the plunger stopper in order to drive the plunger stopper. The recess provides an enhanced flexibility to the plunger stopper.

In one embodiment the body of the plunger stopper comprises at least one of the compounds of the group comprising rubber, e.g. halogenbutyl-rubber-mixture, silicone compounds, thermoplastic elastomers, EPDM rubber (ethylene propylene diene monomer (M-class) rubber), polyurethane compounds, polyolefins and cycloolefins. Usually the plunger stopper is formed as a cylindrical and/or conical body, for example a cylindrical body with a conical section at one or both ends, wherein the surface of the body showing into the direction of the medicament formulation may be either even or tapered (pointed).

The inventors have proven that a silicone-free coating reduces aggregation and formation of particles. Additionally, as break loose force forms the main barrier for controlled plunger stopper movement within the housing of the packaging container, the inventors found out that reduction of break loose forces is another key factor for reliable packaging container handling providing exact doses of medicament formulation.

According to another embodiment the silicone-free coating comprises at least one of the compounds of the group comprising PTFE (polytetrafluoroethylene), ETFE (ethylenetetrafluorethylene), PVF (polyvinyl fluoride), PVDF (polyvinylidene fluoride), PCTFE (polychlorotrifluoroethylene), PFA (perfluoroalkoxy-polymer) and FEP (perfluoro(ethylenepropylene)), wherein in one embodiment the one compound or more compounds of this group has (have together) a proportion of more than 50 wt% of the coating. In another embodiment the one compound or more compounds of this group has (have together) a proportion of more than 80 wt% of the coating. In one embodiment the layer thickness of the silicone-free coating is between 20 µm and 100 µm. These compounds on one hand act as a barrier against elastomeric compounds of the plunger stopper body. On the other hand, they reduce break loose forces and minimize the risk of impurities and drug product degradation with an effective barrier against extractables and leachables that reduces absorption/adsorption of medicament formulation. The inventive packaging container shows reduced variability and less outliers with respect to break loose and gliding forces and hence improved performance in devices such as pen-type drug delivery devices, autoinjectors or pumps. Additionally they provide no or reduced aging effects regarding break loose and gliding force performance in devices. Further, the above coating reduces gliding forces as well so that a reliable drug delivery from the packaging container is provided according to the limits of official authorities.

According to the invention the packaging housing comprises glass, for example the packaging housing may fully consist of glass or the packaging housing may consist of glass and a full or partial coating at its inner surface and/or its outer surface. Glass has superior barrier properties, in particular with regard to oxygen and water, and is also easy to handle during the manufacturing process. In particular, no development and implementation of new manufacturing / filling processes is needed. Established manufacturing equipment can be utilized. According to the invention the glass comprises a silicone coating at at least a part of its inner surface which is bound at the inner surface of the housing by annealing, for example at 280°C to 300°C.

According to another embodiment of the present invention the above object is solved by a system comprising the above described packaging container and a formulation within the packaging container containing one or more pharmaceutically active compound and/or carrier. The packaging container according to the present invention can be used for a medicament formulation that shows incompatibilities or instabilities in contact with silicon. This also applies for packaging containers comprising the siliconized inner surface of the housing as the silicone is bounded at the inner surface due to the annealing step.

In particular, the formulation contains one or more of the following pharmaceutically active compound and/or carrier:
human Insulin, or a human insulin analogue or one of its derivatives, wherein the insulin analogue is Gly(A21), Arg(B31), Arg(B32) human insulin, Lys(B3), Glu(B29) human insulin, Lys(B28), Pro(B29) human insulin, Asp(B28) human insulin, Ala(B26) human insulin, Des(B28-B30) human insulin, Des(B27) human insulin, Des(B30) human insulin, or human insulin, wherein proline in position B28 is replaced by Asp, Lys, Leu, Val or Ala and wherein in position B29 Lys may be replaced by Pro, and wherein Insulin derivatives include B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin; Lysozyme, Factor VIII, β-lactoglobulin (drug carrier), recombinant human growth hormone, albutropin, darbepoetin alfa, keratinocyte growth factor 2 (KGF-2), β-casein, Ribonuclease A, bovine serum albumin (drug carrier), concanavalin A, bevacizumab, ranibizumab, albinterferon α2b, abatacept, adalimumab, monomeric anti-streptavidin IgG1, any immunoglobulin, GLP-1-Agonist.

The above object is further solved by a drug delivery device comprising the above-mentioned system, for example a pen type drug delivery device, an autoinjector or a drug delivery device comprising a pump drive such as a durable pump or a patch pump. The drug delivery device with the pump drive may further comprise a device housing, a plunger rod, a primary container with a cannula (needle assembly), a plunger stopper and/or a flanged cap with a sealing disc.

According to one embodiment, the drug delivery device comprises an expelling mechanism (or dispensing mechanism) configured to displace the plunger stopper in order to expel the medicament formulation from the packaging container.

According to a another embodiment the drug delivery device is a reusable or disposable device for selecting and dispensing a number of user variable or single doses of the medicament formulation, comprising a housing, a packaging container holder for retaining a packaging container containing the medicament, a piston rod displaceable relative to the packaging container holder, a driver coupled to the piston rod, a display member for indicating a set dose and being coupled to the housing and to the driver, and a button coupled to the display member and to the driver, for example rotationally coupled to the display member and to the driver. The expelling mechanism may comprise the piston rod, the driver, the button and/or the display member. The piston rod is adapted to drive the plunger stopper in order to expel the medicament from the packaging container in order to administer the medicament contained in the packaging container. In one embodiment, the housing and the packaging container holder may be one-piece component. In one embodiment a needle or a needle hub may be attached to the distal end of the packaging container holder. In an embodiment the driver is in threaded engagement with the piston rod, permanently rotationally locked to the button, axially displaceable relative to the button and comprises at least two separate components which are rotationally coupled during dose setting and during dose dispensing and which are rotationally decoupled during resetting of the device. Decoupling of the two driver components during resetting has the benefit that both, the piston rod, which is in threaded engagement with the driver, and a dose limiter mechanism, which usually acts on the driver, can be reset together by spinning one of the driver components whereas the other remains stationary in the device. The driver may comprise a third component for coupling the first and second components during dose setting and dose dispensing. The drug delivery device may further comprise a clutch for rotationally coupling the driver to the housing or the display member. In this embodiment the packaging container holder and the housing may be decoupled during resetting in order to replace the empty packaging container.

A non-limiting, exemplary embodiment of the invention will now be described with reference to the accompanying drawings, in which:
- Figure 1: shows a drug delivery device with a system and a cap attached in accordance with the present invention in a side view;
- Figure 2: shows the drug delivery device of Figure 1 with the cap removed and a dose of 79 units dialed;
- Figure 3: shows the components of the drug delivery device of Figure 1 in an exploded view;
- Figure 4: shows a system with a packaging container in accordance with the present invention in a perspective view;
- Figures 5 and 6: examples of plunger stoppers of the packaging container in a longitudinal section;
- Figure 7: shows another drug delivery device with a system in accordance with the present invention in a longitudinal section; and
- Figures 8 and 9: experimental results comparing the break loose and gliding forces for a prior art system (dashed line) and a system according to the invention (solid line) printed as a function of the gliding distance shortly after manufacturing of the respective system (Figure 8) and after 6 months of storage at 5 °C (Figure 9).

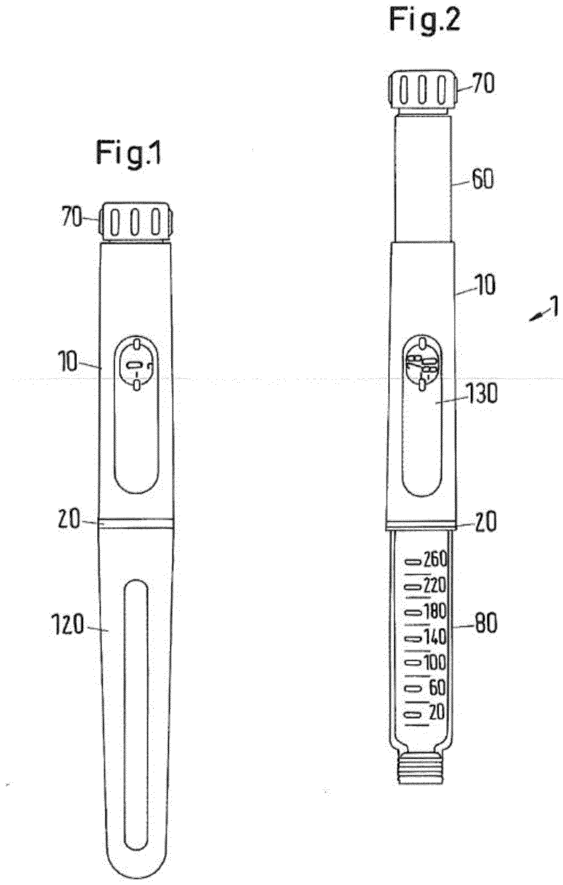

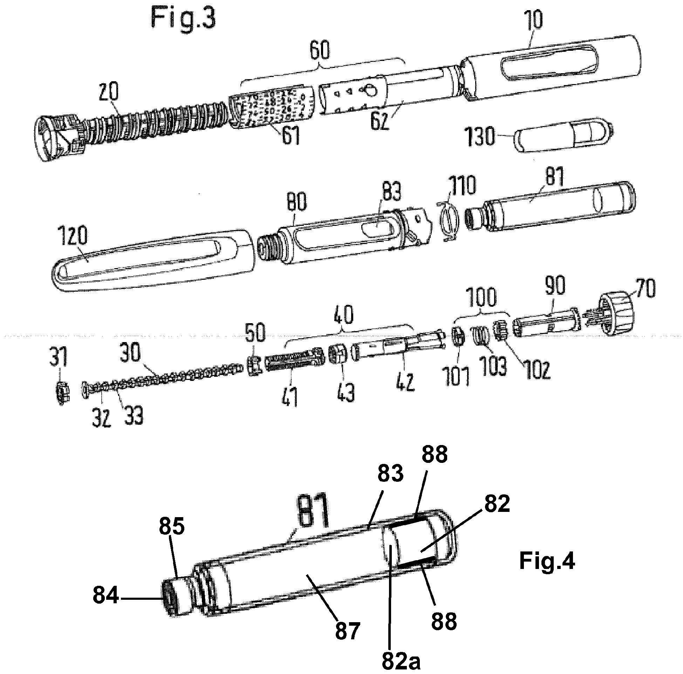

Figures 1 and 2 show a drug delivery device 1 in the form of an injection pen. The device has a distal end (lower end in Figure 1) and a proximal end (upper end in Figure 1). The component parts of the drug delivery device 1 are shown in Figure 3 in more detail. The drug delivery device 1 comprises an outer housing part 10, an inner body 20, a piston rod 30, a driver 40, a nut 50, a display member 60, a button 70, a cartridge holder 80 for receiving a packaging container in form of a cartridge 81, a clutch 90, a clicker 100, a spring 110, a cap 120 and a window insert 130. A needle arrangement (not shown) comprising a needle hub and a needle cover may be provided as additional components, which can be exchanged as explained above. The piston rod 30 comprises a bearing 31. The driver comprises a distal driver part 41, a proximal driver part 42 and a coupler 43. The display member 60 comprises a number sleeve 61 and a dial sleeve 62. The clicker comprises a distal clicker part 101, a proximal clicker part 102 and a spring 103.

The outer housing part 10 is a generally tubular element having a distal part for attaching the inner body 20. Further, an aperture is provided for receiving window insert 130. The outer body 10 provides the user with a surface to grip and react against during dispense.

The inner body 20 is a generally tubular element having different diameter regions. The inner body 20 is received in the outer body 10 and permanently fixed therein to prevent any relative movement of the inner body 20 with respect to the outer body 10. The inner body has the functions to house the drive mechanism within, guiding the clickers and the last dose nut 50 via internal splines, to provide an internal thread through which the piston rod 30 (lead screw) is driven, to support and guide the number sleeve 61 and the dial sleeve 62 on an external thread form, to secure the cartridge holder 80 and to secure the outer body 10 and the window insert 130.

The outermost diameter of the inner body 20 also forms part of the visual design and remains visible when the cap 120 is secured to the cartridge holder 80 as a ring separating the cap 120 from the outer body 10. This visible ring also has depressions which align with the cap snap features on the cartridge holder 80 to indicate that the cartridge holder has been correctly fitted.

Bayonet features on the inner body 20 guide the cartridge holder 80 into the mechanism during cartridge replacement, compressing the cartridge bias spring 110, and then back off the cartridge holder 80 a small distance in order to reduce axial play in the mechanism. Snap features inside the inner body 20 lock the cartridge holder 80 rotationally when it has been correctly fitted. The profile of these snaps aims to prevent the user from partially fitting the cartridge holder 80, the cartridge bias spring 110 ejecting the cartridge holder 80 if the snaps have not at least started to engage. A window retention nose retains the window insert 130 when the outer body 10 and window insert 130 assembly is axially inserted onto the inner body 20. Two diametrically opposite stop faces define the rotational end position for the number sleeve 61. This end position is the end of dose detent position for the minimum dose (0U).

The piston rod 30 is an elongate element having two external threads 32, 33 with opposite hand which overlap each other. One of these threads 32 engages the inner thread of the inner body 20. A disk-like bearing 31 is provided at the distal end of the piston rod 30. The bearing 31 may be a separate component as shown in Figure 3 or may be attached to the piston rod 30 as a one-piece component via a predetermined breaking point.

The piston rod 30 transfers the dispense load from the driver 40 to the bearing 31, creating a mechanical advantage greater than 1:1 by converting the torque generated on the piston rod 30 by the driver 40 thread interface into additional axial load as the piston rod passes through the thread in the inner body 20. The piston rod 30 is reset by pressing on the bearing 31 and this in turn rotates the piston rod back into the inner body 20. This disengages and then rotates the distal drive sleeve 41, resetting the last dose nut 50 back to its starting position on the distal drive sleeve 41.

The driver 40 is a generally tubular element having in the embodiment shown in the Figures three components 41, 42, 43. The distal drive sleeve 41 engages with the piston rod thread 33 to drive the piston rod 30 through the inner body 20 during dose delivery. The distal drive sleeve 41 is also permanently connected to the coupler 43 which in turn is releasably engaged through reset clutch features to the proximal drive sleeve 42. The two halves of the drive sleeve are rotationally and axially connected during dialing and dispense, but are decoupled rotationally during device reset so that they can rotate relative to each other.

The external thread of the distal part 41 engages with the last dose nut 50. Stop faces at the distal part 41 engage with mating stop faces on the last dose nut 50 to limit the number of units that can be dialed.

The proximal drive sleeve 42 shown in Figure 10 supports the clicker components 100 and the clutch 90 and transfers rotational movement from the dose button 90 to the coupler 42 and distal drive sleeve 41.

The coupler 43 rotationally couples the two halves of the driver 40 together during dialing and dispense, whilst allowing them to de-couple during reset.

The last dose nut 50 is provided between the inner body 20 and the distal drive sleeve 41 of driver 40. Stop faces are located on the proximal face of last dose nut 50 to limit the number of units that can be dialed if the stop faces contact stops of distal drive sleeve 41. The function of the last dose nut 50 is to prevent the user from dialing beyond a finite amount. This limit is based on the dispensable volume of the cartridge 81 and when reached, the user must replace the cartridge 81 and reset the device.

The display member 60 is a generally tubular element which is composed of number sleeve 61 and dial sleeve 62 which are snapped together during assembly to axially and rotationally constrain these two components, which thus act as a single part.

The dial sleeve 62 is assembled to the number sleeve 61 such that once assembled, no relative movement is allowed. The parts are made as separate components to enable both molding and assembly.

The button 70 serves as a dose dial grip and is retained by the clutch 90 to transfer the actions of the user to the clutch. It may also carry ratchet teeth that engage a ratchet arm on the dial sleeve 62, which serves as the dispensing clicker giving audible feedback (ratchet clicks), and an end face which serves as the dose completion stop face with the outer body 10. This end face thus serves to define the end position during dispense when it contacts the outer body 10 to provide a very positive stop improving dose accuracy.

The cartridge holder 80 attaches to the inner body 20 with a bayonet connection 82 and houses the ampoule or cartridge 81 containing the medication to be dispensed. The cartridge holder 80 may include an aperture in the rear face which if gripped by the user prevents the ampoule from falling out when the cartridge holder is removed from the inner body 20. The front face is printed with a dose number scale. The threaded distal end is used to attach disposable pen needles.

A tubular clutch 90 is provided between the display member 60 and the button 70. The clutch is fixed relative to and retains the button 70 and together they travel axially relative to the proximal drive sleeve 42 when the button 70 is depressed during dispense, disengaging the clutch teeth from the dial sleeve 62. It also transfers torque from the button to the proximal drive sleeve 42, and the dialing and 0U/80U stop loads from the button via the clutch teeth to the dial sleeve and number sleeve.

The clicker 100 comprises a distal clicker part 101, a proximal clicker part 102 and a spring 103. The clutch spring 103 serves to bias the button 70 out so that at the end of a dose the button 70 pops out, re-engaging the clutch 90 with the dial sleeve 62 ready for dialing. Further, it provides the spring force for the clicker components to act as clickers and also as detent positions for the number sleeve 61. In addition, it holds the two halves of the drive sleeves 41, 42 in rotational engagement during dialing and dispense, whilst allowing them to disengage during device reset.

The distal clicker part 101 is permanently splined to the proximal drive sleeve 42 and engages with the proximal clicker part 102 which in turn is splined to the inner body 20. During dialing when the drive sleeve is rotated relative to the inner body, the two clickers 101, 102, rotate relative to each other under the compression force of the clutch spring 103. This force combined with the clicker teeth formed on the end face of each clicker provides the clicks and also the detent dialing positions.

During dispense the two clickers 101, 102 are pressed together under the dispense load and therefore prevent relative rotation between the proximal drive sleeve 42 and inner body 20, driving the piston rod forwards to deliver the dose.

The cartridge bias spring 110 is assembled as two components one after the other, the lower first and the upper second. The spring combination serves to apply an end load to the cartridge 81 at extremes of tolerance so as to bias it forwards onto the end face of the ferrule in the cartridge holder 80. This ensures that when the user removes and attaches a needle, the friction between the needle cannula and septum of the cartridge does not move the cartridge 81 axially relative to the cartridge holder 80. The bias spring 110 also acts to provide a force against which the user has to connect the cartridge holder 80 and this may add to the tactile feedback of this bayonet joint. The spring 110 also serves to eject the cartridge holder 80 if the cartridge holder is not rotated into a secure position, highlighting this error to the user.

The cartridge 81 is a generally tubular element comprising a plunger stopper 82 at its proximal end which is movable within a tubular housing 83 of the cartridge 81 and closes the housing 83 at its proximal end. At its distal end the housing 83 is closed by a seal 84 which may be pierced by a needle (not shown) when the, preferably double-ended, needle is attached at the distal end of the cartridge holder 80. At its distal end, often the cartridge housing 83 has a neck portion 85 which fits into the threaded distal end of the cartridge holder 80. Between the seal 84 and the plunger stopper 82 the cartridge 81 comprises an inner volume filled with a formulation 87 containing a pharmaceutical active compound and/or carrier as indicated above.

The cartridge housing 83 is made of glass with silicone coating at its inner surface which is annealed at 280°C to 300°C in order to bind the silicone at the surface of the housing 83. Alternatively, the glass housing 83 may have no silicone coating at its inner surface.

The plunger stopper 82 has e.g. a cylindrical form as shown in Figure 4 and is made of a halogenbutyl-rubber-mixture. The plunger stopper 82 comprises a coating 88 at its lateral surface which is in contact with the inner surface of the housing 83. The coating 88 is silicone-free and comprises, for example, more than 50 wt% PTFE and/or ETFE. The layer thickness of coating 88 is between 20 µm and 100 µm. The plunger stopper 82 may have this coating 88 at the surface 82a of the second conical section 82C which is in contact with the medicament formulation 87 within the inner volume as well.

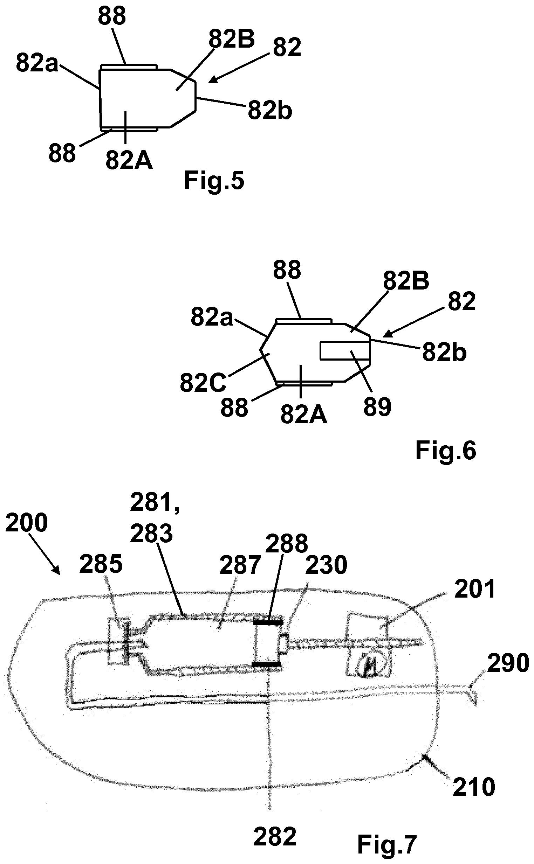

The plunger stopper 82 may have a conical form at least in a section of the plunger stopper. For example, as shown in Figure 5 the plunger stopper 82 comprises a cylindrical section 82A and a conical section 82B. The silicone-free coating 88 is provided at the outer surface of the cylindrical section 82A. The plunger stopper 82 shown in Figure 5 may have this coating 88 at the surface 82a which is in contact with the medicament formulation 87 within the inner volume and which forms an end face of the volume as well. The surface 82a is opposite to the conical section 82B.

Figure 6 shows another embodiment of the plunger stopper 82 comprising a cylindrical center section 82A, a first conical section 82B and a second conical (tapered) section 82C. The first conical section 82B forms the surface 82b which comes in contact with the driving element of the drug delivery device (piston rod 30 with bearing 31) similar to the embodiment shown in Figure 5 whereas the second conical section 82C forms the surface 82a which is in contact with the medicament formulation 87 within the inner volume. The silicone-free coating 88 is provided at the outer surface of the cylindrical section 82A. The plunger stopper 82 shown in Figure 6 may have this coating 88 at the surface 82a as well. The embodiment shown in Figure 6 further comprises a cylindrical recess 89 within the surface 82b of the first conical section 82A. The recess 89 may comprise a thread at its inner surface.

The plunger stoppers shown in Figures 5 and 6 may comprise at least one lamella (lateral contact section) as explained above, for example, at the lateral surface of the cylindrical center section 82A or the (first) conical section 82B. In one embodiment the whole cylindrical center section 82A itself forms the lamella. If the (first) conical section 82B comprises one or several lamellae, in one embodiment, the lamella(e) is(are) located at the distal end of this section. The lamella(e) of the plunger stopper at least partially comprise(s) the silicone free coating 88 at its (their) surface.

In another embodiment the plunger stopper 82 may be formed analogously to the embodiment shown in Figure 6 but with a section 82B which is cylindrical rather than conical.

The cap 120 serves to protect the cartridge holder 80 from damage and the cartridge 81 itself from dust dirt ingress on to the area around the septum. The cap is designed to accommodate a standard pen injector needle.

The window insert 130 may include a lens to magnify the dose numbers e.g. by approximately 25% from their printed size. The window insert 130 may be back printed to protect the printed surface from abrasion and also to maximize the light entering through the window aperture, giving uniform illumination of the dose numbers and white area around these numbers. Arrows may be printed adjacent to the window aperture that indicate the dose dialed.

In the following, the function of the drug delivery device and its components will be explained in more detail. For further information regarding the drug delivery device it is referred to WO 2014/033195 A1, included herein by reference.

To use the device, a user has to select a dose. In the start (at rest) condition as shown in Figures 1 and 2 the display member 60 indicates the number of doses dialed to the user. The number of dialed units can be viewed through the dose window 130 in the outer body 10. Due to the threaded engagement between the display member 60 and the inner body 20 rotation of the button 70 in a clockwise fashion causes the display member 60 to wind out of the device and incrementally count the number of units to be delivered.

During dose setting button 70, driver 40 and display member 60 are rotationally locked together via clutch 90. Further, button 70, driver 40 and display member 60 are axially coupled. Thus, these three components wind out of the outer housing 10 during dose setting. Clockwise rotation of the button 70 causes the driver 40 to rotate and in doing so it advances along the piston rod 30 which remains fixed throughout dialing. The clicker arrangement 100 provides tactile and audible feedback to the user when dialing doses. At the maximum settable dose of 80 units, the stop features of the outer housing part 10 and of the dial sleeve 62 engage to prevent further dialing.

The last dose nut 50 provides the function of counting the number of dispensed units. The nut 50 locks the device at the end of cartridge life and as such no more drug can be dialed by the user. The last dose nut 50 and the driver 40 are connected via a threaded interface as explained above. Further, the last dose nut 50 is assembled into splines such that the nut 50 and the inner body 20 are rotationally locked together (at all times). Rotation of the driver 40 during dialing causes the nut 50 to advance along the outer thread of the distal part. The nut 50 is free to slide axially within the inner body 20 at all times which allows advancement of the nut. At the end of life condition, stop features of the last dose nut 50 contact the corresponding features on the driver 40. The splined contact with inner body 20 reacts any torque transmitted by these stop features.

With the desired dose dialed, the device 1 is ready for dose dispensing. This basically requires pushing button 70 which will result in a disengagement of the clutch 90 from dial sleeve 62 thus allowing relative rotation between the display member 60 and the button 70. In all conditions the driver 40 and the button 70 are rotationally locked together by engagement of arms, fingers and by splines engaging corresponding splines on proximal drive sleeve 42. Thus, with the clutch 90 disengaged (button 70 pushed in) button 70 and driver 40 are rotationally locked together with the button 70, the driver 40 and the display member 60 still being axially coupled.

When dispensing a dose, the dose button 70 and clutch 90 are moved axially relative to the mechanism compressing the clutch spring 103. Because the proximal clicker part 102 is splined to the inner body 20 and the axial load passing through clicker teeth locks the distal clicker part 101 in rotation to the proximal clicker part 102, the mechanism is forced to move axially whilst the dial sleeve 62 and number sleeve 61 are free to spin back into the outer housing 10. The interaction of mating threads between the piston rod 30, driver 40 and inner body 20 delivers a mechanical advantage of, for example, 2:1. In other words, axially advancing driver 40 causes the piston rod 30 to rotate which due to the threaded engagement of piston rod 30 with the inner body 20 advances the piston rod. The piston rod 30 drives the plunger stopper 82 of the cartridge 81 into distal direction so that the medicament formulation 87 containing the one or more pharmaceutically active compound and/or carrier is ejected from the needle attached at the distal end of the cartridge holder 80. Due to the superior break loose and gliding force properties of the plunger stopper 82 (caused by the silicone-free coating 88) the plunger stopper 82 moves easy and constant without bucking. During dose dispensing a dispense clicker is active which involves button 70 and display member 60. The dispense clicker provides primarily audible feedback to the user that drug is being dispensed. Better performance results are especially achieved for stored and aged samples. For the conventional primary packaging system the break loose and gliding forces of the plunger stopper 82 get higher for aged cartridges 81, whereas for the primary packaging system according to the present invention the break loose and gliding forces of the plunger stopper 82 of the aged cartridge 81 remain at same values as for the new cartridge 81.

At this point the dose is complete and when the user removes the force from the end of the dose button 70, the clutch spring 103 pushes this dose button 70 rearwards, re-engaging the teeth between the clutch and the dial sleeve.

Resetting the device starts with removal of the cartridge holder 80 and replacing an empty cartridge with a full cartridge 81. As the cartridge holder is re-attached, the plunger stopper 82 of the new cartridge contacts bearing 31, thus pushing piston rod 30 back into the housing. Initially, the piston rod 30 screws into the inner body 20, thereby axially disengaging the coupler 43 from the proximal drive sleeve 42 against the biasing force of spring 103. Once disengaged the coupler 43 is free to start rotating together with distal drive sleeve 41 and continues to do so as the cartridge holder 80 is moved axially into engagement with the inner body 20.Thus, the distal drive sleeve 41 rotates with respect to the proximal drive sleeve 42 which is still rotationally constrained in inner body 20 as clicker parts 101 and 102 are pressed together by compressed spring 103. As the distal drive sleeve 41 rotates, last dose nut 50 is reset to its (distal) start position. Coupling the cartridge holder 80 to inner body 20 backs off the mechanism due to the bayonet structure 23 allowing re-engagement of the proximal drive sleeve 42 with coupler 43 and thus the distal drive sleeve 41. Now, an injection may be provided using the new cartridge.

Figure 7 shows another embodiment of a drug delivery device, namely a pump device 201. The pump device 201 comprises a device housing 210, a pump drive 201, a plunger rod 230, a primary container 281 comprising a cannula 290 (needle assembly), a plunger stopper 282 and a flanged cap 285 with a sealing disc.

The primary container 281 is similar to the cartridge 81 of the injection pen 1 which is described above, wherein the sealing disc of the flanged cap 285 is similar to the seal 84. In particular, the primary container 281 comprises a formulation 287 containing a pharmaceutical active compound and/or carrier as indicated above and a container housing 283. Further, the construction of the container housing 283 and of the plunger stopper 282 with a coating 288 and their functioning is similar to the respective elements (housing 83, plunger stopper 82) of the cartridge 82 of the injection pen 1 including all above described embodiments of the injection pen and the plunger stopper 82. Accordingly, it is referred to the explanations above with regard to the injection pen 1 and the plunger stopper 82.

For administration of medicine (formulation 287) the prefilled primary container 281 is inserted into the housing 210 of the pump device 200. The housing 210 is typically made of plastic material. After insertion of the primary container 281 into the pump device 200, the sealing disc is pierced by the cannula 290 (needle assembly) and the medicament formulation 287 is administered by energizing the pump drive 201 electrically or actuating the pump drive 201 manually (by pressing at the housing) in order to advance the plunger rod 230 (similar to the piston rod 30 of the injection pen 1) thereby driving the plunger stopper 282. The dosing is provided e.g. by pushing a button at the device housing 210. The operation of the plunger stopper 282, the primary container 281 and the cannula 290 (needle assembly) of the pump device 200 is similar to respective elements of the above described injection pen 1.

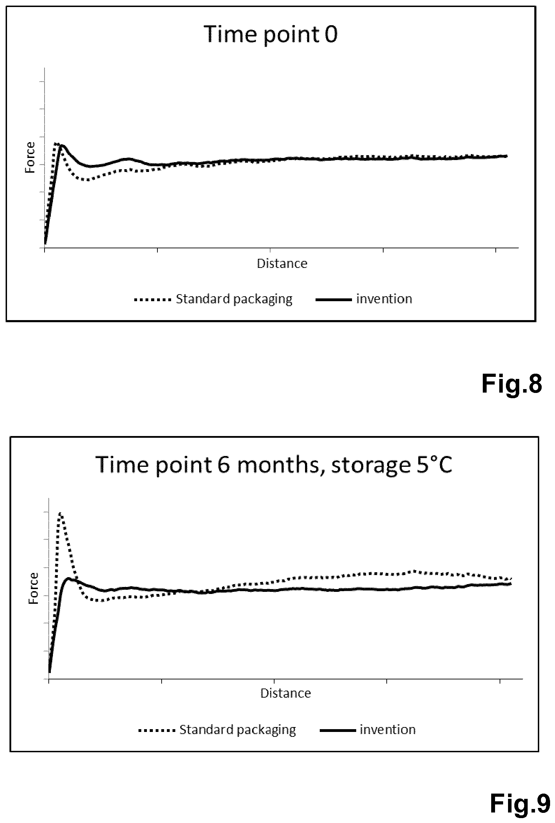

With regard to Figures 8 and 9 a performance test of the inventive system with regard to a prior art system is explained. The applied test procedure is according to DIN EN ISO 11608-3:2001-05.

The prior art system (standard packaging) is a Lantus^{®} 3 ml cartridge. The prior art system comprises a 3 ml cylindrical cartridge made of siliconized pharmaceutical glass type I (65 to 72 wt% SiO₂, 5 to 9 wt% Na₂O, less than or equal to 4 wt% CaO, 14 wt% B₂O₃ and 8 to 13 wt% other components). The plunger stopper of the prior art system has a cylindrical form made of Bromobutyl type I, with a silicone coating on all surfaces of the plunger stopper. The cap of the prior art cartridge is composed of an aluminium layer and an integrated laminate sealing disc comprising bromobutyl and polyisopren.

The prior art system is compared with the inventive system having a 3 ml siliconized glass cartridge, a chlorobutyl plunger stopper with a silicone-free flurotec coating, and a cap with a laminated sealing disk. The silicone-free coating comprises more than 50 wt% PTFE and has a thickness of approximately 50 µm. The plunger stopper has basically the form of the stopper shown in Fig. 6 but does not comprise the cylindrical recess 89. The silicone-free coating covers the surfaces 82a of the second conical section 82C which is in contact with the medicament formulation, the outer surface of the cylindrical section 82A and a distal part of the lateral surface of the first conical section 82B. The silicone-free coating does not cover the proximal end of the lateral surface of the first conical section 82B which is in contact with the driving element of the drug delivery device. A punching edge located at the proximal end of the first conical section 82B is not covered with the silicone-free coating. Along the cylindrical section 82A and the first conical section 82B there are two lamellae at the lateral surface of the plunger stopper both covered with the silicone-free coating. The inventive system cartridge contains Lantus^{®} as the prior art system.

The Lantus^{®} composition is in both cases the composition used in the year 2017.

The performance test was conducted with 20 samples for the prior art system and the inventive system using Ametek^{®} Lloyd instrument LF plus. The measuring length was 41 mm. As a needle cannula in both cases a BD Micro-Fine Ultra 0.33 x 12.7 mm was used.

Fig. 8 shows a comparison of the performance after manufacturing (time point 0) of the prior art system and the inventive system. It can be derived from the diagram, that the break loose force for both systems is approximately the same. At a short distance the gliding force for the prior art system is slightly reduced, whereas at a longer distance the gliding force is the same for both systems.

Fig. 9 depicts the performance test of the systems after 6 month storage of the 20 samples for each system at 5 °C. The performance test was conducted with 20 samples for the prior art system and the inventive system using Ametek^{®} Lloyd instrument LF plus. The measuring length was 41 mm. As a needle cannula in both cases a BD Micro-Fine Ultra 0.33 x 12.7 mm was used. The diagram shows that the break loose force of the prior art system is considerably higher than the break loose force of the inventive system. At short distances, the gliding force of the prior art system is slightly reduced compared with the inventive system, whereas at long distances, the gliding force of the inventive system is lower than the gliding force of the prior art system.

## Claims

1. A packaging container (81, 281) comprising a tubular housing (83, 283) and a plunger stopper (82, 282) moveable within the housing (83, 283), wherein at least a section of an outer surface of the plunger stopper (82, 282) being in contact with an inner surface of the housing (83, 283) comprises a silicone-free coating (88, 288) which reduces break loose and/or gliding forces, **characterised in that** the packaging container housing (83, 283) comprises glass, and **in that** the packaging container housing (83, 283) comprises a coating at least at a part of its inner surface comprising silicone, which is bound at the inner surface of the housing by annealing.

2. The packaging container of claim 1, wherein the coating (88, 288) of the plunger stopper (82, 282) comprises at least one of the compounds of the group comprising PTFE, ETFE, PVF, PVDF, PCTFE, PFA and FEP.

3. The packaging container of any of claims 1 and 2, wherein the silicone-free coating is additionally provided at at least a second section of an outer surface of the plunger stopper wherein the second section faces an inner volume of the packaging container.

4. The packaging container of any of the preceding claims, wherein the plunger stopper (82, 282) has a cylindrical form and/or a conical form.

5. The packaging container of any of the preceding claims, wherein the plunger stopper (82) comprises a recess (89) within its surface opposite the inner volume of the packaging container.

6. The packaging container of any of the preceding claims, wherein the packaging container is suitable for enclosing a formulation (87, 287), wherein the formulation contains one or more pharmaceutically active compound and/or carrier.

7. The packaging container of any of the preceding claims, wherein the packaging container comprises a seal (84) located at the end of the housing (83, 283) which is opposite to the plunger stopper (82, 282), wherein the seal is pierceable, for example by a needle, for discharging the formulation (87, 287).

8. The packaging container of any of the preceding claims, wherein the packaging container is one of a cartridge, a pre-filled syringe, or the like.

9. A system comprising the packaging container (81, 281) of any of the preceding claims and the formulation (87, 287) within the packaging container containing one or more pharmaceutically active compound and/or carrier.

10. The system of claim 9, wherein the formulation (87, 287) contains one or more of the following pharmaceutically active compound and/or carrier:
human Insulin, or a human insulin analogue or one of its derivatives, wherein the insulin analogue is Gly(A21), Arg(B31), Arg(B32) human insulin, Lys(B3), Glu(B29) human insulin, Lys(B28), Pro(B29) human insulin, Asp(B28) human insulin, Ala(B26) human insulin, Des(B28-B30) human insulin, Des(B27) human insulin, Des(B30) human insulin, or human insulin, wherein proline in position B28 is replaced by Asp, Lys, Leu, Val or Ala and wherein in position B29 Lys may be replaced by Pro, and wherein Insulin derivatives include B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin; Lysozyme, Factor VIII, β-lactoglobulin (drug carrier), recombinant human growth hormone, albutropin, darbepoetin alfa, keratinocyte growth factor 2 (KGF-2), β-casein, Ribonuclease A, bovine serum albumin (drug carrier), concanavalin A, bevacizumab, ranibizumab, albinterferon α2b, abatacept, adalimumab, monomeric anti-streptavidin IgG1, any immunoglobulin, GLP-1-Agonist.

11. A drug delivery device (1, 200) comprising the system of any of the claims 9 to 10.

12. The drug delivery device (1, 200) of claim 11, wherein the drug delivery device (1, 200) comprises an expelling mechanism configured to displace the plunger stopper in order to expel the formulation (87, 287) from the packaging container.

## Patentansprüche

1. Verpackungsbehälter (81, 281), umfassend ein röhrenförmiges Gehäuse (83, 283) und einen im Gehäuse (83, 283) bewegbaren Kolbenstopfen (82, 282), wobei wenigstens ein Abschnitt einer in Kontakt mit einer Innenfläche des Gehäuses (83, 283) stehenden Außenfläche des Kolbenstopfens (82, 282) eine siliconfreie Beschichtung (88, 288) umfasst, durch die Abreiss- und/oder Gleitkräfte verringert werden, **dadurch gekennzeichnet, dass** das Verpackungsbehältergehäuse (83, 283) Glas umfasst und dass das Verpackungsbehältergehäuse (83, 283) eine Silicon umfassende Beschichtung wenigstens an einem Teil seiner Innenfläche umfasst, die an die Innenfläche des Gehäuses durch Annealing gebunden wird.

2. Verpackungsbehälter nach Anspruch 1, wobei die Beschichtung (88, 288) des Kolbenstopfens (82, 282) wenigstens eine der Verbindungen der Gruppe umfassend PTFE, ETFE, PVF, PVDF, PCTFE, PFA und FEP umfasst.

3. Verpackungsbehälter nach einem der Ansprüche 1 und 2, wobei die siliconfreie Beschichtung zusätzlich an wenigstens einem zweiten Abschnitt einer Außenfläche des Kolbenstopfens bereitgestellt wird, wobei der zweite Abschnitt einem Innenvolumen des Verpackungsbehälters zugewandt ist.

4. Verpackungsbehälter nach einem der vorhergehenden Ansprüche, wobei der Kolbenstopfen (82, 282) eine zylindrische und/oder eine konische Form aufweist.

5. Verpackungsbehälter nach einem der vorhergehenden Ansprüche, wobei der Kolbenstopfen (82) eine Ausnehmung (89) in seiner dem Innenvolumen des Verpackungsbehälters gegenüberliegenden Oberfläche umfasst.

6. Verpackungsbehälter nach einem der vorhergehenden Ansprüche, wobei der Verpackungsbehälter zum Umschließen einer Formulierung (87, 287) geeignet ist, wobei die Formulierung einen oder mehrere pharmazeutische Wirkstoffe und/oder Träger enthält.

7. Verpackungsbehälter nach einem der vorhergehenden Ansprüche, wobei der Verpackungsbehälter einen Verschluss (84) umfasst, der sich an dem Ende des Gehäuses (83, 283) befindet, das dem Kolbenstopfen (82, 282) gegenüberliegt, wobei der Verschluss zum Ablassen der Formulierung (87, 287) durchstechbar ist, beispielsweise mit einer Nadel.

8. Verpackungsbehälter nach einem der vorhergehenden Ansprüche, wobei es sich bei dem Verpackungsbehälter um entweder eine Kartusche, eine Fertigspritze oder dergleichen handelt.

9. System, umfassend den Verpackungsbehälter (81, 281) nach einem der vorhergehenden Ansprüche und die einen oder mehrere pharmazeutische Wirkstoffe und/oder Träger enthaltende Formulierung (87, 287) im Verpackungsbehälter.

10. System nach Anspruch 9, wobei die Formulierung (87, 287) einen oder mehrere der folgenden pharmazeutischen Wirkstoffe und/oder Träger enthält:
Humaninsulin oder ein Humaninsulinanalog oder eines seiner Derivate, wobei es sich bei dem Insulinanalog um Gly(A21), Arg(B31), Arg(B32)-Humaninsulin, Lys(B3), Glu(B29)-Humaninsulin, Lys(B28), Pro(B29)-Humaninsulin, Asp(B28)-Humaninsulin, Ala(B26)-Humaninsulin, Des(B28-B30)-Humaninsulin, Des(B27)-Humaninsulin, Des(B30)-Humaninsulin oder Humaninsulin handelt, wobei Prolin in Position B28 durch Asp, Lys, Leu, Val oder Ala ersetzt ist und wobei in Position B29 Lys durch Pro ersetzt sein kann und wobei Insulinderivate B29-N-Myristoyl-des(B30)-Humaninsulin; B29-N-Palmitoyl-des(B30)-Humaninsulin; B29-N-Myristoyl-Humaninsulin; B29-N-Palmitoyl-Humaninsulin; B28-N-Myristoyl-LysB28ProB29-Humaninsulin; B28-N-Palmitoyl-LysB28ProB29-Humaninsulin; B30-N-Myristoyl-ThrB29LysB30-Humaninsulin; B30-N-Palmitoyl-ThrB29LysB30-Humaninsulin; B29-N-(N-Palmitoyl-Y-glutamyl)-des(B30)-Humaninsulin; B29-N-(N-Lithocholyl-Y-glutamyl)-des(B30)-Humaninsulin; B29-N-(ω-Carboxyheptadecanoyl)-des(B30)-Humaninsulin und B29-N-(ω-Carboxyheptadecanoyl)-Humaninsulin; Lysozym, Faktor VIII, β-Lactoglobulin (Arzneistoffträger), rekombinantes HGH (Human Growth Hormone), Albutropin, Darbepoetinalfa, KGF-2 (Keratinocyte Growth Factor 2), β-Casein, Ribonuklease A, Rinderserumalbumin (Arzneistoffträger), Concanavalin A, Bevacizumab, Ranibizumab, Albinterferon α2b, Abatacept, Adalimumab, monomeres Anti-Streptavidin-IgG1, jegliches Immunglobulin, GLP-1-Agonist.

11. Medikamenten-Verabreichungsvorrichtung (1, 200), umfassend das System nach einem der Ansprüche 9 bis 10.

12. Medikamenten-Verabreichungsvorrichtung (1, 200) nach Anspruch 11, wobei die Medikamenten-Verabreichungsvorrichtung (1, 200) einen zur Verschiebung des Kolbenstopfens ausgelegten Ausstoßmechanismus umfasst, um die Formulierung (87, 287) aus dem Verpackungsbehälter auszustoßen.

## Revendications

1. Récipient d'emballage (81, 281) comprenant un boîtier tubulaire (83, 283) et un bouchon-piston (82, 282) déplaçable dans le boîtier (83, 283), dans lequel au moins une section d'une surface extérieure du bouchon-piston (82, 282) qui est en contact avec une surface intérieure du boîtier (83, 283) comprend un revêtement sans silicone (88, 288) qui réduit des forces de détachement et/ou de glissement, **caractérisé en ce que** le boîtier (83, 283) de récipient d'emballage comprend du verre, et **en ce que** le boîtier (83, 283) de récipient d'emballage comprend un revêtement au moins au niveau d'une partie de sa surface intérieure comprenant une silicone, qui est lié au niveau de la surface intérieure du boîtier par recuit.

2. Récipient d'emballage selon la revendication 1, dans lequel le revêtement (88, 288) du bouchon-piston (82, 282) comprend au moins un des composés du groupe comprenant PTFE, ETFE, PVF, PVDF, PCTFE, PFA et FEP.

3. Récipient d'emballage selon l'une quelconque des revendications 1 et 2, dans lequel le revêtement sans silicone est fourni de plus au moins au niveau d'une deuxième section d'une surface extérieure du bouchon-piston, dans lequel la deuxième section fait face à un volume intérieur du récipient d'emballage.

4. Récipient d'emballage selon l'une quelconque des revendications précédentes, dans lequel le bouchon-piston (82, 282) a une forme cylindrique et/ou une forme conique.

5. Récipient d'emballage selon l'une quelconque des revendications précédentes, dans lequel le bouchon-piston (82) comprend un renfoncement (89) dans sa surface opposée au volume intérieur du récipient d'emballage.

6. Récipient d'emballage selon l'une quelconque des revendications précédentes, dans lequel le récipient d'emballage est approprié pour contenir une formulation (87, 287), dans lequel la formulation contient un ou plusieurs composés et/ou supports pharmaceutiquement actifs.

7. Récipient d'emballage selon l'une quelconque des revendications précédentes, dans lequel le récipient d'emballage comprend un joint d'étanchéité (84) situé au niveau de l'extrémité du boîtier (83, 283) qui est opposée au bouchon-piston (82, 282), dans lequel le joint d'étanchéité peut être percé, par exemple par une aiguille, pour décharger la formulation (87, 287).

8. Récipient d'emballage selon l'une quelconque des revendications précédentes, dans lequel le récipient d'emballage est l'une parmi une cartouche, une seringue préremplie ou similaire.

9. Système comprenant le récipient d'emballage (81, 281) selon l'une quelconque des revendications précédentes et la formulation (87, 287) dans le récipient d'emballage contenant un ou plusieurs composés et/ou supports pharmaceutiquement actifs.

10. Système selon la revendication 9, dans lequel la formulation (87, 287) contient un ou plusieurs du/des composé et/ou support pharmaceutiquement actifs suivants
insuline humaine, ou analogue d'insuline humaine ou l'un de ses dérivés, dans lequel l'analogue d'insuline est l'insuline humaine Gly(A21), Arg(B31), Arg(B32), l'insuline humaine Lys(B3), Glu(B29), l'insuline humaine Lys(B28), Pro(B29), l'insuline humaine Asp(B28), l'insuline humaine Ala(B26), l'insuline humaine Des(B28-B30), l'insuline humaine Des(B27), l'insuline humaine Des(B30) ou une insuline humaine dans laquelle la proline en position B28 est remplacée par Asp, Lys, Leu, Val ou Ala et dans laquelle en position B29 Lys peut être remplacée par Pro, et dans lequel les dérivés de l'insuline comprennent l'insuline humaine B29-N-myristoyl-des(B30) ; l'insuline humaine B29-N-palmitoyl-des(B30) ; l'insuline humaine B29-N-myristoyl ; l'insuline humaine B29-N-palmitoyl ; l'insuline humaine B28-N-myristoyl LysB28ProB29 ; l'insuline humaine B28-N-palmitoyl-LysB28ProB29 ; l'insuline humaine B30-N-myristoyl-ThrB29LysB30 ; l'insuline humaine B30-N-palmitoyl-ThrB29LysB30 ; l'insuline humaine B29-N-(N-palmitoyl-Y-glutamyl)-des(B30) ; l'insuline humaine B29-N-(N-lithocholyl-Y-glutamyl)-des(B30) ; l'insuline humaine B29-N-(ω-carboxyheptadécanoyl)-des(B30) et l'insuline humaine B29-N-(ω-carboxyheptadécanoyl) ; lysozyme ; facteur VIII, β-lactoglobuline (support de médicament), hormone de croissance humaine recombinante, albutropine, darbépoétine alfa, facteur de croissance des kératinocytes 2 (KGF-2), β-caséine, ribonucléase A, albumine sérique bovine (support de médicament), concanavaline A, bevacizumab, ranibizumab, albinterféron α2b, abatacept, adalimumab, IgG1 anti-streptavidine monomérique, toute immunoglobuline, agoniste de GLP-1.

11. Dispositif d'administration de médicament (1, 200) comprenant le système selon l'une quelconque des revendications 9 à 10.

12. Dispositif d'administration de médicament (1, 200) selon la revendication 11, dans lequel le dispositif d'administration de médicament (1, 200) comprend un mécanisme d'expulsion configuré pour déplacer le bouchon-piston afin d'expulser la formulation (87, 287) du récipient d'emballage.
